(19) Europäisches Patentamt
European Patent Office
Office européen des brevets

(11) Numéro de publication: **0 352 167 A1**

(12) # DEMANDE DE BREVET EUROPEEN

(21) Numéro de dépôt: **89401970.2**

(22) Date de dépôt: **10.07.89**

(51) Int. Cl.$^5$: **C 07 C 69/76**
C 07 C 67/36

(30) Priorité: **20.07.88 FR 8809792**

(43) Date de publication de la demande:
**24.01.90 Bulletin 90/04**

(84) Etats contractants désignés:
**AT BE CH DE ES FR GB GR IT LI LU NL SE**

(71) Demandeur: **RHONE-POULENC CHIMIE**
**25, quai Paul Doumer**
**F-92408 Courbevoie Cédex (FR)**

(72) Inventeur: **Huser, Marc**
**36, rue du Fossé Rietberg**
**F-67100 Strasbourg (FR)**

**Osborn, John**
**10, rue Daniel Hirtz**
**F-67000 Strasbourg (FR)**

(74) Mandataire: **Tavernier, Colette et al**
**RHONE-POULENC CHIMIE Service Brevets Chimie 25,**
**Quai Paul Doumer**
**F-92408 Courbevoie Cédex (FR)**

(54) **Procédé d'alcoxycarbonylation de derives aromatiques halogenes.**

(57) La présente invention concerne un procédé d'alcoxycarbonylation mettant en oeuvre un nouveau catalyseur constitue d'un complexe palladium-phosphine, celle-ci présentant un pKa supérieur ou égal à 7, un dérivé aromatique chloré, du monoxyde de carbone et un alcool.

EP 0 352 167 A1

## Description

# PROCEDE D'ALCOXYCARBONYLATION DE DERIVES AROMATIQUES HALOGENES

La présente invention concerne un procédé d'alcoxycarbonylation. Elle concerne plus particulièrement l'alcoxycarbonylation de dérivés aromatiques chlorés en présence d'un catalyseur à base de palladium.

Il est connu par exemple selon le brevet US 3988358 de procéder à l'alcoxycarbonylation de composés aromatiques, vinyliques ou hétérocycliques halogénés à une température d'environ 20-150° C sous environ 1 à 100 atmosphères en présence d'un catalyseur à base d'un complexe du palladium de formule $PdX_2$ $[P(C_6H_5)_3]_{1 \text{ ou } 2}$ dans laquelle X représente un halogène ou le groupe acétate et en présence d'une amine.

Il est précisé dans le texte que la triphenyl phosphine peut être remplacée par la trianisylphosphine, la tri p.tolylphosphine, la tri-n butylphosphine, la triéthylphosphine... L'ensemble des essais a été réalisée sur la triphénylphosphine qui de nos jours est la phosphine la plus couramment utilisée pour ce type de réaction. L'inconvénient de cette phosphine est qu'elle ne permet que l'alcoxycarbonylation de dérivés bromés ou iodés aromatiques et jamais l'alcoxycarbonylation de dérivés chlorés. Or les dérivés aromatiques chlorés sont beaucoup moins chers que les dérivés bromés il serait donc avantageux de pouvoir les utiliser comme matière de départ pour la réaction d'alcoxycarbonylation.

La présente invention a permis d'atteindre cet objectif. Elle a pour objet un procédé d'alcoxycarbonylation en milieu homogène de dérivés aromatiques chlorés en présence d'un catalyseur à base de palladium.

Le catalyseur à base de palladium est précisément choisi parmi les complexes du palladium et d'une phosphine. Cette phosphine doit présenter un pKa supérieur à 7, tel que défini par WmA HENDERSON, Jr ; C.A. STREULI dans le journal of American Chemical Society, 82, 1960, 5791.

On peut citer parmi cette classe de phosphines non limitativement :
- la tricyclohexyl phosphine,
- la triisopropyl phosphine,
- la triéthyl phosphine,
- la tri n-butyl phosphine,
- la tritertiobutyl phosphine,
- la dicyclohexylphényl phosphine.

Parmi cette classe de phosphines présentant un pKa supérieur à 7, on préfère utiliser les phosphines qui présentent un angle de cône tel que défini par C.A. TOLMAN, dans le Journal of American Chemical Society, 92, 1970, 2956 compris entre 160 et 180°.

Font partie de ce domaine préférentiel les phosphines suivantes
- la tricyclohexyl phosphine
- la triisopropyl phosphine
- la dicyclohexyl phényl phosphine

On préfère tout particulièrement utiliser la tricyclohexylphosphine.

Le complexe de la présente invention répond à la formule (I) suivante :

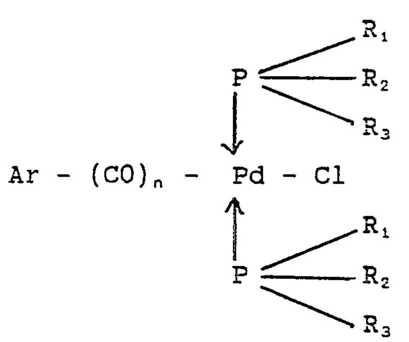

(I)

dans laquelle
- $R_1$, $R_2$, $R_3$ représentent chacun des groupes identiques ou différents choisis parmi les radicaux cyclohexyle, isopropyle, l'un des groupes $R_1$, $R_2$ ou $R_3$ pouvant être remplacé par un groupe phényl lorsque les deux autres représentent un groupe cyclohexyle,
- Ar représente un radical aromatique mono, polycyclique ou hétérocyclique.
- n est égal à 0 ou 1.

Le complexe de formule (I) précédemment décrit est notamment utilisé pour catalyser les réactions d'alcoxycarbonylation selon le mécanisme réactionnel suivant :

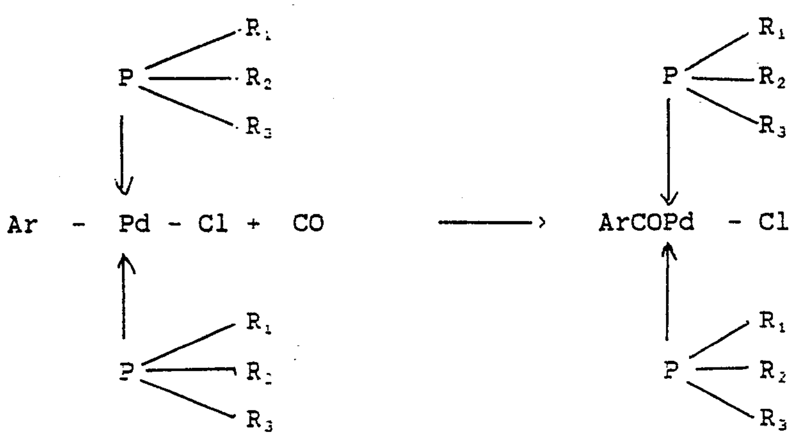

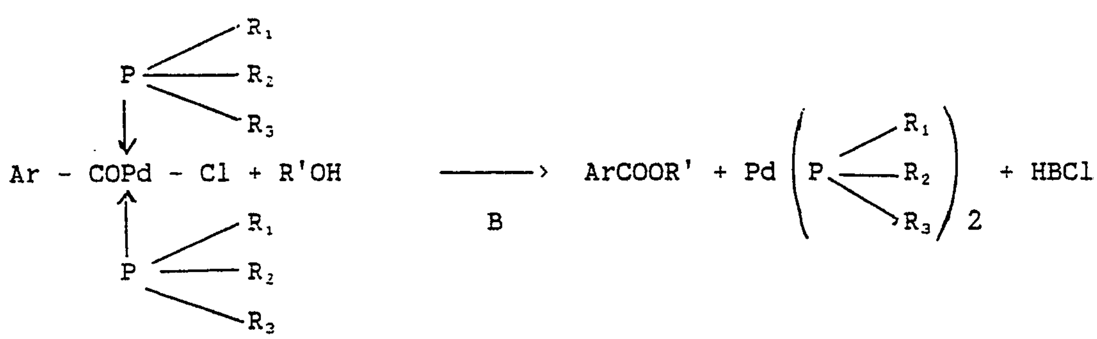

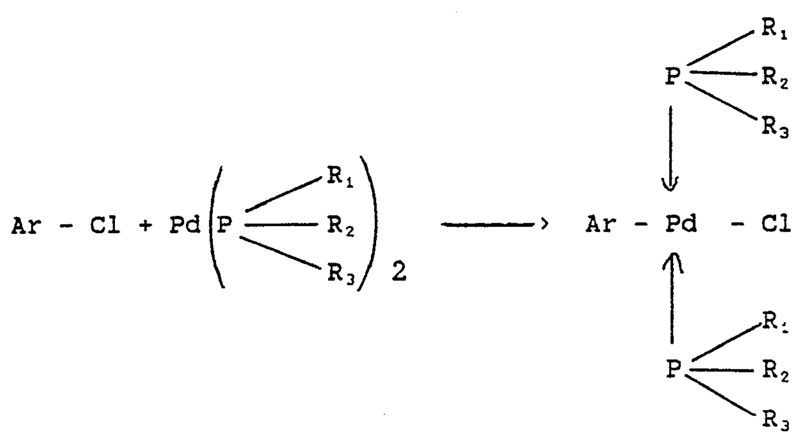

qui d'une manière simplifiée peut être résumée par l'équation suivante :

$$R'OH + ArCl + CO \xrightarrow[B]{\text{catalyseur à base de Pd}} ArCOOR' + Cl\ B\ H$$

Dans les équations précédentes, le terme "R" signifie soit un groupe cyclohexyl, soit un groupe phényl, soit un groupe isopropyl. Le phosphore peut être ligandé à 3 groupes équivalents comme dans la tricyclohexyl phosphine ou par des groupes différents comme dans la dicyclohexylphényl phosphine.

R'OH représente un alcool aliphatique ou aromatique contenant de préférence 1 à 12 atomes de carbone.

Le dérivé aromatique chloré (ArCl) peut être mono, polycyclique ou hétérocyclique. Il peut être éventuellement substitué par un groupe alkoxy, alkyle, alkylcarbonyle, cycloalkyle, cycloalkoxy, halogéno, halogénoalkyle, halogénoalkoxy, aryle, aryloxy, halogénoaryle, halogénoaryloxy, alkylaryle, aralkyle, alkylcarbonyloxy, cycloalkylcarbonyloxy.

Les chaînes alkyles des divers groupes alkyle ou alkoxy contiennent de préférence 1 à 6 atomes de carbone, les groupes aryle contiennent de préférence 6 à 18 atomes de carbone.

On préfère utiliser les dérivés aromatiques monocycliques non substitués ou substitués par un groupe alkoxy, contenant 1 à 6 atomes de carbone, alkyle contenant 1 à 6 atomes de carbone, chloro, fluoro, alkyl carbonyle dont la chaîne alkyle contient 1 à 6 atomes de carbone.

Parmi les dérivés aromatiques chlorés utilisables dans le procédé de l'invention, on peut citer à titre illustratif :
- le chlorobenzène
- les dichlorobenzènes
- les chlorofluorobenzènes
- les chlorotoluènes
- les chloroanisoles
- les chloronaphtalènes
- les méthyl, éthyl, propylchlorobenzoates
- la méthylchlorophénylcétone
- les chlorobiphényles
- le chloroindole
- le chlorothiophène

Une base (B) est nécessaire pour neutraliser l'acide chlorhydrique formé au cours de la réaction d'alcoxycarbonylation. Cette base peut être constituée de la phosphine elle-même ou d'une base différente. Si cette base est différente de la phosphine, elle a de préférence un pKa supérieur à celui de la phosphine de manière à ce que cette dernière ne joue pas inutilement le rôle de base neutralisante.

Elle doit de préférence être soluble dans le milieu réactionnel. On préfère utiliser les trialkylamines et par exemple la triéthylamine, la triisopropylamine, la tri-n butylamine. Les bases minérales peuvent aussi être utilisées telles que le carbonate de sodium mais n'apportent pas d'avantage particulier.

Le solvant utilisé pour la mise en oeuvre de l'invention est choisi parmi les solvants aromatiques hydrocarbonés tels que :
- le toluène
- les xylènes

les éthers, tels que :
- le dioxane

les alcools, tels que :
- l'éthanol
- l'isopropanol

les cétones, telles que :
- la méthylisobutylcétone

les nitriles, tels que :
- le benzonitrile

les amides, tels que :
- le diméthylformamide

Les réactifs tels que le dérivé chloro aromatique ou la base peuvent servir de milieu réactionnel.

Le complexe de formule I peut être utilisé tel quel comme catalyseur.

Le complexe de formule I peut être aussi formé "in situ" par au moins trois méthodes de mise en oeuvre.

Selon une première méthode de mise en oeuvre du procédé de l'invention, on met en contact un composé de formule (II) suivante :

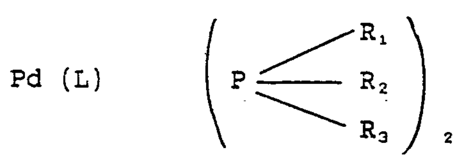

( II )

dans laquelle
- le motif L représente un groupe labile en présence de ArCl.
- les groupes $R_1$, $R_2$, $R_3$ ont la même signification que dans la formule (I) avec un dérivé halogéno aromatique de formule ArCl du monoxyde de carbone et l'alcool dans un solvant.

Selon une deuxième méthode de mise en oeuvre du procédé de l'invention on met en présence un complexe de palladium à l'état d'oxydation zéro tel que :

Pd $(L)_3$, au moins deux équivalents de phosphine répondant à la formule

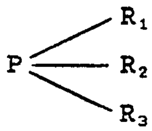

avec le dérivé chloroaromatique de formule ArCl du monoxyde de carbone et l'alcool.

Selon une troisième méthode de mise en oeuvre du procédé de l'invention, on met en présence un sel de palladium à l'état d'oxydation II choisi par exemple parmi le dichlorure, le dibromure, le diiodure de palladium, le diacétate de palladium, le nitrate de palladium, le sulfate de palladium, l'oxyde de palladium avec le dérivé chloroaromatique du monoxyde de carbone, l'alcool et au moins deux équivalents de phosphine de formule

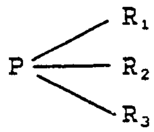

Au sens de la présente invention, on entend par groupe labile (L) tout groupe qui en présence de ArCl peut être facilement échangeable.

Parmi ces groupes, on peut citer non limitativement :
- la dibenzylidène acétone (DBA)
- les groupes alkylène et de préférence l'éthylène

Lorsque l'on part d'un complexe du palladium ne contenant pas de phosphine (deuxième ou troisième méthode de mise en oeuvre), on préfère utiliser au moins 2 mol de phosphine par atome gramme de palladium et de préférence entre 2 et 10000 mol et tout particulièrement entre 2 et 5.

On préfère utiliser une quantité de solvant telle que la concentration en complexe ou en sel de palladium dans le milieu soit comprise entre $10^{-5}$ et 100 mmol par litre.

La concentration minimale en base doit correspondre à la stoéchiométrie de la réaction. Elle peut être utilisée en quantité nettement supérieure et même être utilisée comme solvant. Il faut qu'en fin de réaction, on n'est pas épuisé la base.

La concentration du dérivé aromatique chloré peut varier dans de larges limites puisqu'il peut être utilisé comme solvant. Il est dans ce cas facilement recyclé.

La concentration de l'alcool peut aussi varier dans de larges limites puisqu'il peur être utilisé comme solvant. Lorsqu'il n'est pas utilisé comme solvant un rapport de 1 à 5 par rapport au dérivé chloroaromatique est tout à fait conseillé.

La température réactionnelle est avantageusement comprise entre 50 et 250° C et de préférence entre 100 et 200° C.

La pression partielle de monoxyde de carbone est notamment comprise entre 1 et 300 bar et de préférence entre 10 et 100 bar.

La présente invention sera plus complètement décrite à l'acide des exemples suivants qui ne doivent pas être considérés comme limitatifs de l'invention.

Dans les exemples suivants les abréviations suivants signifient:

$PCy_3$ = tricyclohexyl phosphine

$$TT = \text{taux de transformation} \quad \frac{\text{quantité de dérivé aromatique halogéné transformée}}{\text{quantité de dérivé aromatique halogéné introduite}} \times 100$$

$$RT = \text{rendement sur produit transformé} \quad \frac{\text{quantité de produit désiré formé (mol)}}{\text{quantité de produit transformé (mol)}} \times 100$$

EXEMPLES 1 et 2

INFLUENCE DE LA NATURE DE LA PHOSPHINE

Dans un réacteur en Hastelloy HB2 ®, on introduit :

. 50 mmol de $C_6H_5Cl$
. 110 mmol de $NEt_3$
. 50 mmol de $CH_3OH$
. q.s.p 30 ml de toluène

On ajoute . 1 matg de Pd $(OAc)_2$
. 5 mmol de $PL_3$

On augmente la température sous une pression de 15 bar de CO jusqu'à 180°C. On laisse la réaction se poursuivre pendant 12 heures.

| Ex | $PL_3$ | TT % | RT($C_6H_5COOMe$) % | RT($C_6H_5COOEt$) % |
|---|---|---|---|---|
| 1 | $PCy_3$ | 30 | 60 | 13 |
| 2 | $PiPr_3$ | 24 | 71 | 8 |
| COMP1 | $PtBu_3$ | 0 | | |
| COMP2 | $PEt_3$ | 0 | | |
| COMP3 | $PBz_3$ | 0 | | |
| COMP4 | $PPh_3$ | 0 | | |

EXEMPLE 3

INFLUENCE DE LA TEMPERATURE

On reproduit l'exemple 1 en modifiant la température de réaction.

| Ex | Température °C | TT % | RT($C_6H_5COOMe$) % | RT($C_6H_5COOEt$) % |
|---|---|---|---|---|
| 1 | 180 | 30 | 60 | 13 |
| 3 | 200 | 54 | 2 | 74 |

EXEMPLES 4 à 7

INFLUENCE D'UNE SUBSTITUTION EN PARA SUR LE CHLOROBENZENE

On reproduit l'exemple 1 en utilisant un dérivé chloroaromatique variable et en ne laissant la réaction se poursuivre que pendant 4 heures.

| Ex | R | RT(R-$C_6H_4COOMe$) % | TT % | Autres produits |
|---|---|---|---|---|
| 1* | H | 73 | 30 | $C_6H_5COOEt$ |
| 4 | F | nd | 13 | |
| 5 | COOMe | nd | 86 | $ClC_6H_4COOEt$, $C_6H_4(COOEt)_2$ $C_6H_4(COOMe)(COOEt)$ |
| 6 | OMe | nd | 12 | |
| 7 | Cl | 46 | 26 | $C_6H_4(COOMe)_2$ |

nd : non dosé ;

* : 12 heures

EXEMPLES 8 à 9

INFLUENCE DE LA NATURE DE L'ALCOOL

On reproduit l'exemple 1 en modifiant l'alcool introduit R'OH et en laissant la réaction se dérouler que pendant 4 heures.

| Ex | R'OH | TT |
|---|---|---|
| 1 | MeOH | 30 |
| 8 | nBuOH | 10 |
| 9 | $C_6H_5CH_2OH$ | 28 |

EXEMPLES 10 et 11

INFLUENCE DE LA NATURE DE LA BASE

On reproduit l'exemple 1 en modifiant la nature de la base.

| Ex | BASE | TT % |
|---|---|---|
| 10 | $NEt_3$ | 30 |
| 11 | pyridine | 0 |

EXEMPLE 12

INFLUENCE DE LA PRESSION DE MONOXYDE DE CARBONE

On reproduit l'exemple 1 en modifiant la pression de CO.

| Ex | Pression CO bar | TT % | RT($C_6H_5COOMe$) % |
|---|---|---|---|
| 1 | 15 | 30 | 73 |
| 12 | 100 | 24 | 66 |

**Revendications**

1. Procédé d'alcoxycarbonylation de composés aromatiques halogénés caractérisé en ce qu'on met en présence un dérivé aromatique chloré, un catalyseur à base de palladium, une phosphine présentant un Pka supérieur à 7 en présence d'une base avec de l'alcool et de l'oxyde de carbone.

2. Procédé selon la revendication 1 caractérisé en ce que le dérivé chloroaromatique répond à la formule ArCl dans laquelle Ar représente un radical aromatique mono, polycyclique ou hétérocyclique éventuellement substitué.

3. Procédé selon la revendication 2 caractérisé en ce que Ar représente un radical benzénique, éventuellement substitué par un groupe alkyle, alkoxy, alkylcarbonyle, cycloalkyle, cycloalkoxy, alkylcarbonyloxy, cycloalkylcarbonyloxy, aryle, aryloxy, arylcarbonyloxy, aryloxycarbonyle ; fluoro, chloro, halogénoalkyle, halogénoalkoxy, halogénocycloalkyle, halogénocycloalkyloxy, halogénoaryle, halogénoaryloxy, les motifs alkyle ou alkoxy ayant de 1 à 12 atomes de carbone.

4. Procédé selon la revendication 3 caractérisé en ce que le composé ArCl est le chlorobenzène, le chloroanisole, l'ester éthylique de l'acide chlorobenzoïque.

5. Procédé selon la revendication 1 caractérisé en ce que la phosphine est choisie parmi les phosphines présentant un angle de cône compris entre 160 et 180°.

6. Procédé selon les revendications 1 et 5, caractérisé en ce que la phosphine est choisie parmi la tricyclohexylphosphine, la triisopropylphosphine, la dicyclohexylphénylphosphine.

7. Procédé selon les revendication 5 et 6, caractérisé en ce que la phosphine est la tricyclohexylphosphine.

8. Procédé selon la revendication 1 caractérisé en ce qu'on ajoute une base choisie parmi les amines tertiaires et les bases minérales en quantité molaire supérieure au dérivé aromatique.

9. Procédé selon la revendication 1 caractérisé en ce que l'alcool est un alcool aliphatique ou benzylique contenant 1 à 12 atomes de carbone.

10. Procédé selon la revendication 1 caractérisé en ce que la réaction a lieu dans un excès de réactif ou en présence d'un solvant choisi parmi les dérivés aromatiques ou aliphatiques hydrocarbonés, eventuellement halogénés, les éthers, les alcools, les cétones, les amides, les nitriles.

11. Procédé selon la revendication 1 caractérisé en ce que la quantité de palladium exprimé en milliatome-gramme de métal noble ou en millimoles de dérivé métallique par litre est comprise dans

l'intervalle $10^{-5}$ à 100.

12. Procédé selon les revendications 1 et 11 caractérisé en ce que la quantité de phosphine est telle que le nombre des atomes-grammes de phosphore au nombre des atomes-grammes de métal soit compris dans l'intervalle 2 à 10000.

13. Procédé selon la revendication 1 caractérisé en ce que la pression réactionnelle est comprise entre 1 et 300 bar et de préférence entre 10 et 100 bar.

14. Procédé selon la revendication 1, caractérisé en ce que la température réactionnelle est comprise entre 50 et 250° C et de préférence comprise entre 100 et 200° C.

15. Procédé de mise en oeuvre caractérisé en qu'on introduit dans un solvant un complexe du palladium de formule (I) suivante

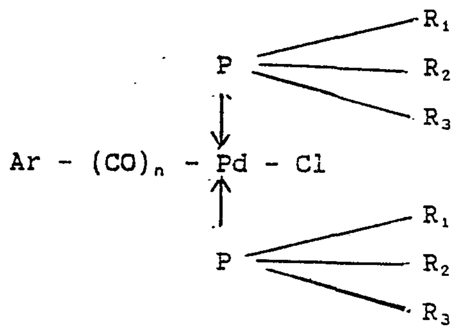

(I)

dans laquelle. $R_1$, $R_2$, $R_3$ représentent chacun des groupes identiques ou différents choisis parmi les radicaux cyclohexyle, isopropyle, l'un des groupes $R_1$, $R_2$ ou $R_3$ pouvant être remplacé par un groupe phényl lorsque les deux autres représentent un groupe cyclohexyle,

. Ar représente un radical aromatique mono, polycyclique ou hétérocyclique

. n est égal à 0 ou 1 avec un dérivé chloroaromatique , du monoxyde de carbone et un alcool en présence éventuellement d'un excès de phosphine.

16. Procédé de mise en oeuvre caractérisé en ce qu'on introduit dans un solvant un complexe du palladium de formule (II) suivante

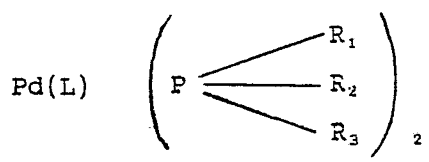

(II)

dans laquelle

. $R_1$, $R_2$, $R_3$ ont la même signification que dans la formule (I)

. L représente la dibenzylidène acétone ou un groupe alkylène avec un dérivé chloroaromatique, du monoxyde de carbone et un alcool en présence éventuellement d'un excès de phosphine.

17. Procédé de mise en oeuvre caractérisé en ce qu'on introduit dans un solvant un complexe de palladium de formule $Pd(L)_3$ dans laquelle L a la même signification que dans la formule (II), un dérivé chloroaromatique, du monoxyde de carbone et un alcool en présence d'une phosphine de formule

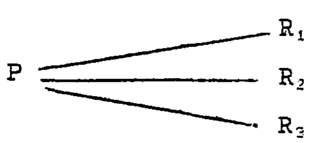

dans laquelle

$R_1$, $R_2$, $R_3$ ayant la même signification que dans la formule (I).

18. Procédé de mise en oeuvre caractérisé en ce qu'on introduit dans un solvant un complexe de palladium à l'état d'oxydation II, un dérivé chloroaromatique, du monoxyde de carbone et de l'alcool en présence d'une phosphine de formule

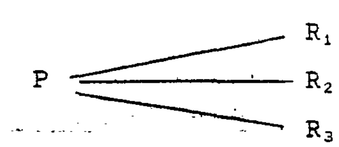

dans laquelle

$R_1$, $R_2$, $R_3$ ayant la même signification que dans la formule (I).

19. Procédé selon la revendication 17 caractérisé en ce que le complexe de palladium à l'état

d'oxydation II est choisi parmi le dichlorure, le dibromure, le diiodure de palladium, le diacétate de palladium, le sulfate de palladium, l'oxyde de palladium.

Office européen des brevets

# RAPPORT DE RECHERCHE EUROPEENNE

Numero de la demande

EP 89 40 1970

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| A | US-A-4 654 436 (D.W. LANE)<br>* Colonne 2, lignes 35-51; colonne 2, ligne 62 - colonne 3, ligne 37 *<br>--- | 1 | C 07 C 69/76<br>C 07 C 67/36 |
| D,A | US-A-3 988 358 (R.F. HECK)<br>* Colonne 2, lignes 3-33; colonne 2, ligne 63 - colonne 3, ligne 6; colonnes 3-4, exemples 1-9; colonnes 7-8, revendications *<br>----- | 1 | |
| | | | **DOMAINES TECHNIQUES RECHERCHES (Int. Cl.5)** |
| | | | C 07 C 69/00<br>C 07 C 67/00 |

Le présent rapport a été établi pour toutes les revendications

| Lieu de la recherche | Date d'achèvement de la recherche | Examinateur |
|---|---|---|
| LA HAYE | 17-10-1989 | KINZINGER J.M. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant